**Europäisches Patentamt**

**European Patent Office**

**Office européen des brevets**

(19)

(11) Veröffentlichungsnummer: **0 640 582 A2**

## (12) EUROPÄISCHE PATENTANMELDUNG

(21) Anmeldenummer: **94112181.6**

(22) Anmeldetag: **04.08.94**

(51) Int. Cl.6: **C07C 67/11**, C07C 69/92, C07C 69/94

(30) Priorität: **31.08.93 DE 4329286**

(43) Veröffentlichungstag der Anmeldung:
**01.03.95 Patentblatt 95/09**

(84) Benannte Vertragsstaaten:
**BE CH DE DK ES FR GB IT LI NL**

(71) Anmelder: **CASSELLA Aktiengesellschaft**
**Hanauer Landstrasse 526**
**D-60386 Frankfurt (DE)**

(72) Erfinder: **Müller, Rolf, Dr.**
**Dornbachweg 3**
**D-61184 Karben-Rendel (DE)**
Erfinder: **Wessel, Thomas, Dr.**
**Wächtersbacher Strasse 11**
**D-60386 Frankfurt am Main (DE)**

(74) Vertreter: **Muley, Ralf, Dr.**
**Cassella AG,**
**Patentabteilung,**
**Hanauer Landstrasse 526**
**D-60386 Frankfurt (DE)**

(54) Verfahren zur Herstellung von aromatischen Methoxycarbonsäuremethylestern.

(57) Die Erfindung betrifft ein Herstellungsverfahren für aromatische Methoxycarbonsäuremethylester durch Umsetzung von aromatischen Hydroxycarbonsäuren im Gemisch mit den Methoxycarbonsäuren mit Dimethylsulfat in Wasser.

EP 0 640 582 A2

Die vorliegende Erfindung betrifft ein Verfahren zur Herstellung von aromatischen Methoxycarbonsäuremethylestern durch eine Methylierungsreaktion mit Dimethylsulfat in wäßriger Lösung.

Aromatische Methoxycarbonsäurester finden als wertvolle Synthesebausteine eine weit verbreitete Anwendung, z.B. als Zwischenprodukte für Pharmazeutika oder als Komponenten von Polyestern. So ist z.B. 6-Methoxy-1-naphthoesäuremethylester ein wichtiges Zwischenprodukt bei der Synthese des Pharmawirkstoffs Tolrestat, eines Medikaments zur Vorbeugung und Behandlung von Spätfolgen des Diabetes mellitus (s. z.B. EP-B-59 596, EP-B-200 840, EP-A-307 519, US-A-4 808 748). Ebenfalls stellt z.B. 3-Methoxy-4-methylbenzoesäuremethylester ein wertvolles Zwischenprodukt für pharmazeutische Präparate dar (s. z.B. EP-B-83 228).

Eine allgemein anwendbare Methode zur Herstellung von Carbonsäureestern ist die Alkylierung der Salze der Carbonsäuren mit Dialkylsulfaten, für die Herstellung der Methylester also die mit Dimethylsulfat, s. z.B. Houben-Weyl-Müller, Methoden der Organischen Chemie, 4. Auflage, Band 8, S.541-543. Ebenso ist die Veretherung von Phenolen mit Dialkylsulfaten ein anerkanntes Verfahren zur Herstellung von Alkoxyaromaten, insbesondere die mit Dimethylsulfat ein Herstellungsverfahren für Methoxyaromaten, s. z.B. Houben-Weyl-Müller, Methoden der Organischen Chemie, 4. Auflage, Band 6/3, S. 62-66.

Die gleichzeitige oder in mehreren Schritten durchgeführte Alkylierung der Hydroxygruppe und der Carboxygruppe in Phenolcarbonsäuren ist, wenn die betreffenden aromatischen Hydroxycarbonsäuren gut zugänglich sind, ein brauchbares Verfahren zur Herstellung von aromatischen Alkoxycarbonsäurealkylestern, insbesondere von Methoxycarbonsäuremethylestern, wobei als Methylierungsmittel auch Dimethylsulfat in Betracht kommt. Umsetzungen von Phenolcarbonsäuren mit Dialkylsulfaten und insbesondere Dimethylsulfat sind z.B. beschrieben im Organikum, 15. Auflage, VEB Deutscher Verlag der Wissenschaften, S.251-253, oder Houben-Weyl-Müller, s.o., Band 6/3, S.62-66. Bei solchen Umsetzungen von Phenolcarbonsäuren mit Dimethylsulfat reagiert die reaktivere phenolische Hydroxygruppe im allgemeinen schneller als die Carboxygruppe. Wenn nur die Hydroxygruppe methyliert werden soll, ist dieses Verfahren häufig akzeptabel, obwohl auch dann Produktgemische die Regel sind.

Soll eine vollständige Methylierung, sowohl an der Hydroxygruppe als auch an der Carboxygruppe, erzielt werden, so sind die bekannten Verfahren für die Überführung von Phenolcarbonsäuren in Methoxycarbonsäuremethylester für eine Durchführung im technischen Maßstab aber im allgemeinen nicht geeignet. Nachteilig ist insbesondere der im allgemeinen sehr große Überschuß an Dimethylsulfat, der für eine weitgehend vollständige Methylierung eingesetzt werden muß, und der nach der Umsetzung aufwendig vernichtet oder abgetrennt werden muß, wobei trotz oder auch wegen des großen Überschusses vorliegende Nebenprodukte oft noch langwierige und kostspielige Produktreinigungen erforderlich machen, so daß die erzielte Ausbeute unökonomisch niedrig ist.

So wird z.B. in der in den Monatsheften für Chemie, Band 91, S.1077 ff. (1960), beschriebenen Methylierung von Gallussäure trotz des Einsatzes der 6,4-fachen molaren Menge an Dimethylsulfat, bezogen auf die Molzahl umzusetzender Hydroxy- und Carboxygruppen, nur eine Ausbeute an vollständigem Methylierungsprodukt von 75 % erzielt. In der in der EP-B-200 840 beschriebenen Herstellung von 6-Methoxy-1-naphthoesäuremethylester wird eine 3,4-fache molare Menge an Dimethylsulfat, bezogen auf die Gesamtmolzahl zu methylierender Hydroxy- und Carboxygruppen in dem eingesetzten, aus der dortigen vorherigen Reaktionsstufe so erhaltenen Gemisch aus 6-Hydroxy-1-naphthoesäure und 6-Methoxy-1-naphthoesäure, verwandt. Das Verfahren liefert aber zudem nur ein Produkt geringer Reinheit (87 %; s. Vergleichsbeispiel 1). In der in den Berichten der Deutschen Chemischen Gesellschaft, Band 37, S. 3658 ff. (1904) erwähnten Methylierung von Hydroxynaphthoesäuren wird mit der 2fachen molaren Menge an Dimethylsulfat, bezogen auf die Molzahl zu methylierender Gruppen, nur eine Ausbeute von 70 % am gewünschten Produkt erhalten.

Bekannte Verfahren, die geringere Überschüsse einsetzen, zeichnen sich durch andere Nachteile aus, die einer Anwendung im technischen Maßstab entgegenstehen oder - aus ökologischen oder Sicherheitsgründen - aufwendige apparatetechnische Vorkehrungen erfordern würden. So ist die in den Berichten der Deutschen Chemischen Gesellschaft, Band 40, S.714 ff. (1907), beschriebene Herstellung von 2-Methoxybenzoesäuremethylester, bei der die Gesamtmengen der Ausgangssubstanzen zusammengegeben und erwärmt werden, wegen der in der Hitze einsetzenden, unkontrollierten Reaktionsweise nicht reproduzierbar und nicht in den technischen Maßstab übertragbar. In der in der US-Patentschrift 4 590 290 beschriebenen Herstellung von 6-Methoxy-1-naphthoesäuremethylester werden zwar nur 1,2 mol Dimethylsulfat pro mol zu methylierender Hydroxy- und Carboxygruppen in der eingesetzten 6-Hydroxy-1-naphthoesäure verwandt, es wird aber in einer Ausbeute von nur 78 % trotz aufwendiger Reinigung ein Produkt mit lediglich 93%iger Reinheit erhalten, und insbesondere wird die Umsetzung in Butylacetat als Lösungsmittel durchgeführt, dessen Verwendung im Produktionsmaßstab umfangreiche Maßnahmen zum Brand- und Explosionsschutz der Anlage und zur Abluftreinigung voraussetzt. Dies gilt auch bei Verwendung der bei Methylierungen beispielsweise eingesetzten Lösungsmittel Dioxan, Aceton oder Methanol. Das in dem Verfahren nach der EP-B-200 840, in der Wasser als Lösungsmittel nur erwähnt, in den Beispielen aber nicht verwandt wird, als Lösungsmittelkomponente verwandte 1,2-Dichlorethan ist darüberhinaus wegen seiner toxikologischen Eigenschaften vor allem unter arbeitshygienischen Aspekten zu vermeiden.

Es besteht somit ein Bedarf nach einem einfachen, im technischen Maßstab durchführbaren, ohne organische Lösungsmittel auskommenden Verfahren zur Herstellung von aromatischen Methoxycarbonsäuremethylestern, bei dem die bei bekannten Methylierungen von aromatischen Hydroxycarbonsäuren eingesetzten unökonomisch hohen Überschüsse an Methylierungsmittel vermieden werden.

Überraschend wurde nun gefunden, daß diese Aufgabe dadurch gelöst wurde, daß aromatische Hydroxycarbonsäuren im Gemisch mit den zugehörigen Methoxycarbonsäuren in Wasser mit der nur 0,8 bis 1,7fachen molaren Menge an Dimethylsulfat, bezogen auf die Gesamtmolzahl zu methylierender Hydroxy- und Carboxygruppen in den Ausgangssubstanzen, umgesetzt werden. Dabei werden unter besonders weitgehender Ausnutzung des Dimethylsulfats in hoher Ausbeute sehr reine Produkte erhalten, ohne daß aufwendige Aufarbeitungs- und Reinigungsschritte erforderlich sind.

Gegenstand der vorliegenden Erfindung ist somit ein Verfahren zur Herstellung von aromatischen Methoxycarbonsäuremethylestern der allgemeinen Formel I,

$$CH_3O\text{-Ar-COOCH}_3 \qquad (I)$$

in der Ar für einen Phenylenrest oder für einen Naphthylenrest steht, wobei der Phenylenrest auch mono- oder disubstituiert und der Naphthylenrest auch mono-, di- oder trisubstituiert sein kann durch Halogenatome, $(C_1\text{-}C_4)$-Alkylgruppen, $(C_2\text{-}C_5)$-Alkenylgruppen, $(C_1\text{-}C_4)$-Alkoxygruppen, Benzyloxygruppen, Trifluormethylgruppen, Nitrogruppen, Hydroxymethylgruppen, Formylgruppen, Cyangruppen und $((C_1\text{-}C_4)\text{-Alkoxy})$carbonylgruppen,

durch Umsetzung von aromatischen Hydroxycarbonsäuren der allgemeinen Formel II

$$HO\text{-Ar-COOH} \qquad (II)$$

im Gemisch mit aromatischen Methoxycarbonsäuren der allgemeinen Formel III,

$$CH_3O\text{-Ar-COOH} \qquad (III)$$

wobei Ar in II und III wie für I angegeben definiert ist, mit Dimethylsulfat in Gegenwart einer Base, dadurch gekennzeichnet, daß man die Umsetzung in Wasser durchführt und daß man die 0,8 bis 1,7fache molare Menge an Dimethylsulfat, bezogen auf die Gesamtmolzahl zu methylierender Hydroxy- und Carboxygruppen in den Ausgangssubstanzen, einsetzt.

Beispiele für Hydroxycarbonsäuren, aus denen im Gemisch mit den zugehörigen Methoxycarbonsäuren nach dem erfindungsgemäßen Verfahren somit Methoxycarbonsäuremethylester hergestellt werden, sind 2-Hydroxy-, 3-Hydroxy- und 4-Hydroxybenzoesäuren und 1-Hydroxy-, 2-Hydroxy-, 3-Hydroxy-, 4-Hydroxy-, 5-Hydroxy-, 6-Hydroxy-, 7-Hydroxy- und 8-Hydroxy-1-naphthoesäuren und -2-naphthoesäuren. Neben der Hydroxy- bzw. Methoxygruppe und der Carboxy- bzw. Methoxycarbonylgruppe kann der Aromat im Falle der Benzoesäurederivate noch zusätzlich ein oder zwei und im Falle der Naphthoesäurederivate noch zusätzlich ein, zwei oder drei weitere Substituenten tragen, wobei sich die Substituenten in beliebigen Positionen befinden können, und im Falle der Naphthoesäurederivate auch auf beide Ringe verteilt sein können und wobei die Substituenten gleich, teilweise gleich oder alle verschieden voneinander sein können.

Beispiele für Halogenatome, die als Substituenten am Benzol- bzw. Naphthalinkern stehen können, sind Fluor, Chlor, Brom und Iod. Beispiele für $(C_1\text{-}C_4)$-Alkylgruppen sind der Methyl-, der Ethyl-, der n-Propyl-, der i-Propyl-, der n-Butyl-, der i-Butyl-, der s-Butyl- und der t-Butylrest, Beispiele für $(C_2\text{-}C_5)$-Alkenylgruppen sind der Vinyl-, der Allyl-, der 2-Methylallyl-, der 2-Butenyl-, der 1,1-Dimethylallyl- und der 4-Methyl-2-butenylrest. Beispiele für $(C_1\text{-}C_4)$-Alkoxygruppen, die als Substituenten auftreten können, sind der Methoxy-, der Ethoxy-, der n-Propoxy-, der i-Propoxy-, der n-Butoxy-, der i-Butoxy- und der t-Butoxyrest. Beispiele für $((C_1\text{-}C_4)\text{-Alkoxy})$carbonylgruppen sind der Methoxycarbonyl-, der Ethoxycarbonyl-, der i-Propoxycarbonyl-, der n-Butoxycarbonyl-, der i-Butoxycarbonyl- und der t-Butoxycarbonylrest.

Bevorzugt umfaßt das erfindungsgemäße Verfahren die Herstellung von aromatischen Methoxycarbonsäuremethylestern der allgemeinen Formel Ia,

$$CH_3O\text{-Ar}^1\text{-COOCH}_3 \qquad (Ia)$$

3

in der Ar$^1$ für einen 3-Phenylenrest oder einen 4-Phenylenrest steht, der keine weiteren Substituenten trägt oder als weitere Substituenten noch eine oder zwei zusätzliche (C$_1$-C$_4$)-Alkylgruppen trägt, aus den entsprechenden 3-Hydroxy- und 4-Hydroxybenzoesäuren im Gemisch mit den Methoxysäuren. Besonders bevorzugt ist die Herstellung von Verbindungen der allgemeinen Formel Ib,

$$CH_3O\text{-}Ar^2\text{-}COOCH_3 \qquad (Ib)$$

in der Ar$^2$ für einen 3-Phenylenrest oder einen 4-Phenylenrest steht, der als weitere Substituenten zwei oder bevorzugt eine zusätzliche Methylgruppe trägt, aus den entsprechenden 3-Hydroxy- und 4-Hydroxybenzoesäuren im Gemisch mit den Methoxysäuren. Darüberhinaus bevorzugt ist die Herstellung von 3-Methoxy-4-methylbenzoesäuremethylester aus 3-Hydroxy-4-methylbenzoesäure im Gemisch mit 3-Methoxy-4-methylbenzoesäure.

Eine andere bevorzugte Ausführungsform des erfindungsgemäßen Verfahrens umfaßt die Herstellung von aromatischen Methoxycarbonsäuremethylestern der allgemeinen Formel Ic,

$$CH_3O\text{-}Ar^3\text{-}COOCH_3 \qquad (Ic)$$

in der Ar$^3$ für einen Naphthylenrest steht, der keine weiteren Substituenten oder als weitere Substituenten noch eine oder zwei zusätzliche (C$_1$-C$_4$)-Alkylgruppen, bevorzugt Methylgruppen, trägt, aus den entsprechenden Hydroxynaphthoesäuren im Gemisch mit den Methoxynaphthoesäuren. Besonders bevorzugt ist die Herstellung von Verbindungen der allgemeinen Formel Id,

$$CH_3O\text{-}Ar^4\text{-}COOCH_3 \qquad (Id)$$

in der Ar$^4$ für einen keine weiteren Substituenten tragenden 1,4- oder 1,5- oder 1,6- oder 1,7- oder 1,8- oder 2,4- oder 2,5- oder 2,6- oder 2,7- oder 2,8-Naphthylenrest steht, aus der entsprechenden Hydroxy-1-naphthoesäure bzw. Hydroxy-2-naphthoesäure im Gemisch mit der Methoxynaphthoesäure. Darüberhinaus bevorzugt ist die Herstellung von 6-Methoxy-1-naphthoesäuremethylester aus 6-Hydroxy-1-naphthoesäure im Gemisch mit der 6-Methoxy-1-naphthoesäure.

Die als Ausgangsmaterial dienenden aromatischen Hydroxycarbonsäuren und Methoxycarbonsäuren sind bekannt oder nach bekannten Herstellverfahren zugänglich. Die 6-Methoxy-1-naphthoesäure beispielsweise ist in Liebig's Annalen der Chemie, Band 188, S.8 (1877), im Journal of the Chemical Society, Band 123, S.1641 (1923) oder in der EP-B-268 088 beschrieben, die 6-Methoxy-1-naphthoesäure im Journal of the American Chemical Society, Band 69, S.2261 (1947). Die 3-Hydroxy-4-methylbenzoesäure ist beispielsweise beschrieben in den Berichten der Deutschen Chemischen Gesellschaft, Band 6, S.481 (1873), die 3-Methoxy-4-methylbenzoesäure in Helvetica Chimica Acta, Band 19, S.370 (1936) oder im Journal of the Chemical Society, Band 119, S.1342 (1921). Das in der erfindungsgemäßen Umsetzung methylierte Säuregemisch kann, außer aus vorher jeweils isoliert vorliegender Hydroxy- und Methoxycarbonsäure erhalten worden zu sein, auch bereits in der vorherigen Synthesestufe als Gemisch angefallen sein, wie z.B. in dem bereits genannten Fall der EP-B-200 840.

Die Menge an Methoxycarbonsäure der allgemeinen Formel III, die bei der Durchführung des erfindungsgemäßen Verfahrens im Gemisch mit der Hydroxycarbonsäure der allgemeinen Formel II mit Dimethylsulfat umgesetzt wird, ist an sich beliebig. Bevorzugt werden Mischungen umgesetzt, die pro Mol Hydroxycarbonsäure der allgemeinen Formel II bis zu 2 mol der zugehörigen Methoxycarbonsäure der allgemeinen Formel III enthalten, also z.B. 0,01 bis 2 mol oder 0,05 bis 2 mol pro Mol Hydroxycarbonsäure, aber z.B. auch 0,01 bis 1 mol oder 0,05 bis 1 mol pro Mol Hydroxycarbonsäure. Welches Verhältnis besonders günstig ist, hängt vom Einzelfall ab. Bei der besonders bevorzugten Herstellung von 6-Methoxy-1-naphthoesäuremethylester setzt man besonders bevorzugt eine Mischung um, die pro Mol 6-Hydroxy-1-naphthoesäure 0,1 bis 1 mol, darüber hinaus bevorzugt 0,3 bis 0,8 mol, 6-Methoxy-1-naphthoesäure enthält. Bei der ebenfalls besonders bevorzugten Herstellung von 3-Methoxy-4-methylbenzoesäuremethylester setzt man besonders bevorzugt eine Mischung um, die pro Mol 3-Hydroxy-4-methylbenzoesäure 0,05 bis 1 mol, darüber hinaus bevorzugt 0,1 bis 0,4 mol, 3-Methoxy-4-methylbenzoesäure enthält.

In einer bevorzugten Ausführungsform stammt die in dem umzusetzenden Carbonsäuregemisch enthaltene Methoxycarbonsäure der allgemeinen Formel III vollständig oder teilweise, besonders bevorzugt vollständig, aus einer oder mehreren vorherigen Umsetzungen nach dem erfindungsgemäßen Verfahren. Je nach den Reaktionsbedingungen, die vom jeweiligen Einzelfall abhängen, wird ein Teil der Carboxygruppen in den eingesetzten Säuren nicht methyliert, außer dem Produkt der vollständigen Methylierung, der Verbindung der allgemeinen Formel I, kann also in unterschiedlicher Menge als Nebenprodukt der Reaktion

EP 0 640 582 A2

auch wieder die Methoxycarbonsäure der allgemeinen Formel III anfallen. Letzere kann von dem Methylester der allgemeinen Formel I leicht nach dem Prinzip der pH-Trennung abgetrennt werden.

Beispielsweise kann zur Aufarbeitung des Reaktionsgemisches erst bei einem geeigneten, im allgemeinen im Alkalischen liegenden pH-Wert, bei dem die Verbindung der allgemeinen Formel III als Salz vorliegt, das vollständig methylierte, kein Salz bildende Produkt der allgemeinen Formel I durch Filtration, Zentrifugation, Abtrennung einer Flüssigphase, Extraktion oder ein anderes übliches, für den Einzelfall geeignetes Verfahren abgetrennt werden, und anschließend beispielsweise durch Ansäuern auf einen geeigneten pH-Wert, bei dem die Verbindung der allgemeinen Formel III als freie Säure vorliegt, die nicht vollständig methylierte Verbindung der allgemeinen Formel III in die leicht isolierbare Form der freien Säure überführt werden und diese dann durch ein geeignetes übliches Verfahren von der wäßrigen Lösung abgetrennt werden. Die wieder isolierte Methoxycarbonsäure der allgemeinen Formel III ist im allgemeinen so rein, daß sie dann ohne weitere Reinigungsoperation bei einem Folgeansatz der zu methylierenden aromatischen Hydroxycarbonsäure der allgemeinen Formel II zugesetzt werden kann.

Die bei einer Reihe von Folgeansätzen nach der bevorzugten Ausführungsform bei jedem Ansatz neben dem vollständig methylierten Produkt isolierte und dem nächsten Ansatz wieder zugesetzte Menge der Methoxycarbonsäure der allgemeinen Formel III hängt von der Struktur der Verbindungen und von den Reaktionsbedingungen ab und kann natürlich in gewissem Maße auch von Ansatz zu Ansatz schwanken, ist aber bei geeigneter Wahl der Dimethylsulfatmenge und der Reaktionsbedingungen weitgehend konstant. Wie oben beschrieben können auch die Hydroxycarbonsäuren der allgemeinen Formel II allein, ohne Zusatz der Methoxycarbonsäuren der allgemeinen Formel III, oder die Methoxycarbonsäuren der allgemeinen Formel III allein, ohne Zusatz der Hydroxycarbonsäuren der allgemeinen Formel II, umgesetzt werden. Wenn eine Folge von Ansätzen nach der bevorzugten Ausführungsform, bei dem die Säure der allgemeinen Formel III aus vorherigen Umsetzungen stammt, ausgehend allein von der Hydroxysäure der allgemeinen Formel II begonnen werden soll, wird dann diese in Wasser in Gegenwart einer Base mit der 0,8 bis 1,7fachen molaren Menge an Dimethylsulfat, bezogen auf die Gesamtmolzahl zu methylierender Hydroxy- und Carboxygruppen, umgesetzt. Entsprechendes gilt, wenn aus der aus dem letzten einer Folge von Ansätzen nach der bevorzugten Ausführungsform isolierten Methoxysäure der allgemeinen Formel III ohne nochmaligen Zusatz neuer Hydroxysäure noch weiterer Methylester der allgemeinen Formel I hergestellt werden soll.

Die Durchführungsweise des erfindungsgemäßen Verfahrens und die Reaktionsbedingungen hängen vom Einzelfall ab. Üblicherweise werden im Reaktionsgefäß Wasser, die Base oder ein Teil der Base und die zu methylierende Substanz, also die Hydroxycarbonsäure der allgemeinen Formel II im Gemisch mit der Methoxycarbonsäure der allgemeinen Formel III vorgelegt, das Reaktionsgemisch wird gegebenenfalls auf die gewünschte Temperatur gebracht, und dann wird in Portionen bzw. kontinuierlich über eine bestimmte, vom Einzelfall abhängige Zeitdauer das Dimethylsulfat bei der gewünschten Temperatur zudosiert und gegebenenfalls auch weitere Base zudosiert, wobei insbesondere auch durch das Zudosieren der Base ein für den jeweiligen Einzelfall besonders günstiger pH-Bereich eingehalten werden kann. Bei dieser Vorgehensweise findet nur eine untergeordnete Hydrolyse des Dimethylsulfats statt. Die Reaktion kann aber beispielsweise auch derart durchgeführt werden, daß die Gesamtmenge oder ein Teil des Dimethylsulfats im Gemisch mit Wasser vorgelegt wird und bei der gewünschten Temperatur über eine bestimmte Zeitdauer das zu methylierende Material und die Base zudosiert werden, beispielsweise in Form einer mit der Base hergestellten wäßrigen Lösung des Salzes der einzusetzenden Carbonsäure, wobei auch hier wieder die Base so zudosiert werden kann, daß ein besonders günstiger pH-Bereich eingehalten wird. Die Reaktion läuft in diesem Fall von Beginn an in einem Zweiphasen-System ab. Gegebenenfalls wird nach Zudosieren der Carbonsäuren weiteres Dimethylsulfat und weitere Base zudosiert. Weiterhin kann die Reaktion beispielsweise auch in einem Durchflußreaktor durchgeführt werden, in dessen Reaktionskammer bei der gewünschten Temperatur sowohl das Dimethylsulfat als auch die zu methylierende Substanz und die Base dosiert werden, wobei auch hier wieder später gegebenenfalls weiteres Dimethylsulfat und Base zum Reaktionsgemisch nachgesetzt werden können. In allen diesen Fällen können durch eine kontrollierte Reaktionsführung definierte, für den Einzelfall besonders günstige Reaktionsbedingungen eingehalten werden. Dadurch ist ein reproduzierbarer hoher Ausnutzungsgrad des Dimethylsulfats gegeben. Je nach Einzelfall kann auch eine Dimethylsulfatmenge von 1,0 bis 1,7 mol oder 1,0 bis 1,6 mol oder 1,0 bis 1,5 mol, bezogen auf die Gesamtmolzahl zu methylierender Hydroxy- und Carboxygruppen in den Ausgangssubstanzen, bevorzugt sein. Im Falle der Herstellung von 6-Methoxy-1naphthoesäuremethylester setzt man bevorzugt die 1,0 bis 1,3fache, besonders bevorzugt die 1,0 bis 1,1fache molare Menge an Dimethylsulfat, bezogen auf die Gesamtmolzahl zu methylierender Hydroxy- und Carboxygruppen in der 6-Hydroxy- und der 6-Methoxy-1-naphthoesäure, ein.

Die Reaktionstemperatur und der pH-Wert, bei denen die erfindungsgemäße Methylierung durchgeführt wird, richten sich nach den durch die Struktur des Substrats bedingten Reaktivitäten der Hydroxy- und Carboxygruppe sowie nach der Hydrolyseneigung der entstehenden Methoxycarbonylgruppe und des Dimethylsulfats und sind somit vom Einzelfall abhängig. Zudem ist die Natur der zugesetzten Base von Einfluß. Bei drucklosem Arbeiten können Reaktionstemperaturen bis zum Siedepunkt des Reaktionsgemischs angebracht sein. Bevorzugt wird die Umsetzung zwischen 10° und 90°C, besonders bevorzugt zwischen Raumtemperatur und 70°C, ganz besonders bevorzugt zwischen 30° und 60°C durchgeführt. Die Temperatur kann auch während der Reaktion geändert werden, beispielsweise gegen Ende der Reaktion erhöht werden. Die Reaktion kann nicht nur drucklos, sondern auch unter Druck, z.B. dem in einem geschlossenen System sich einstellenden Eigendruck, durchgeführt werden.

Je nach Einzelfall kann die gesamte Menge der Base zu Beginn der Reaktion vorgelegt werden und der pH-Wert im folgenden unbeeinflußt bleiben, oder es kann anfänglich ein Teil der Base vorgelegt und dann später durch weitere Basenzugabe ein bestimmter pH-Bereich eingehalten werden, oder es kann auch während des gesamten Reaktionsverlaufs unter pH-Kontrolle gearbeitet werden. Dabei kann es, um optimale Umsetzungsgrade und Ausnutzungsgrade des Dimethylsulfats zu erzielen, angebracht sein, während des Reaktionsablauf den pH-Bereich zu ändern, beispielsweise erst einen Teil des Dimethylsulfats in einem höheren und dann einen weiteren Teil in einem niedrigeren pH-Bereich zuzudosieren, aber ebenso kann die umgekehrte Vorgehensweise angebracht sein. Im allgemeinen wird die Reaktion im alkalischen Bereich durchgeführt, aber je nach der Struktur des Substrats können auch teilweise schwach saure pH-Werte angebracht sein. In einer Ausführungsweise des erfindungsgemäßen Verfahrens sind bei der Herstellung von 6-Methoxy-1-naphthoesäuremethylester während der Phase der pH-Kontrolle beispielsweise pH-Werte zwischen 8 und 12 günstig.

Als Basen kommen für die Verwendung im erfindungsgemäßen Verfahren alle die in Betracht, die unter den Reaktionsbedingungen keine störenden Nebenreaktionen eingehen, insbesondere solche, die durch Dimethylsulfat nicht methyliert werden. Bevorzugt werden anorganische Basen eingesetzt, aber es können beispielsweise auch tetrasubstituierte Ammoniumhydroxide, sterisch gehinderte Amine, Alkoholate, wie Natrium- oder Kaliummethylat, -ethylat oder -tert-butylat, oder geeignete Ionenaustauscher eingesetzt werden. Besonders bevorzugt sind Alkali- und Erdalkalimetallhydroxide und -carbonate, wobei als Erdalkali- und Alkalimetalle insbesondere Calcium, Barium, Lithium, Natrium, Kalium und Caesium in Frage kommen. Darüberhinaus bevorzugt sind Alkalimetallhydroxide, wie Lithium-, Natrium-, Kalium- oder Caesiumhydroxid, von diesen vor allem Natrium- und Kaliumhydroxid und ganz besonders wiederum Kaliumhydroxid. Vorteilhaft kann auch die Verwendung von Gemischen mehrerer Basen sein, z.B. von Natriumhydroxid zusammen mit Kaliumhydroxid oder von Kaliumcarbonat zusammen mit Kaliumhydroxid. Die Base oder die Basen können in reiner Form oder handelsüblicher, als Feststoff, oder in Form wäßriger Lösungen eingesetzt werden. Bei der Auswahl der Base sind auch die Löslichkeitseigenschaften der im Reaktionsgemisch vorliegenden Salze zu berücksichtigen. Die Menge der einzusetzenden Base hängt vom jeweiligen Einzelfall ab und ist z.B. durch die Wahl des oder der pH-Bereiche, in denen die Reaktion durchgeführt wird, beeinflußt. Es werden insgesamt zumindest soviel Mol an Basenäquivalenten benötigt, daß die Gesamtmolzahl an Hydroxy- und Carboxygruppen in den umzusetzenden Hydroxy- und Methoxycarbonsäuren deprotoniert werden kann, im allgemeinen wird aber eine darüber hinausgehende Menge an Base zugesetzt.

Mögliche Aufarbeitungsarten des bei der Durchführung des erfindungsgemäßen Verfahrens anfallenden Reaktionsgemisches wurden bereits erwähnt. Die Aufarbeitung kann nach an sich bekannten Isolierungs- bzw. Trennmethoden erfolgen, beispielsweise durch Filtration, Zentrifugation, Phasentrennung, Extraktion, (Vakuum-)Destillation oder Wasserdampfdestillation auch durch chromatographische Methoden, insbesondere unter Ausnutzung des Prinzips der pH-Trennung. Durch Zugabe einer Base nach Beendigung der Reaktion kann ein geeigneter pH-Wert eingestellt werden, bei dem die Methoxycarbonsäure der allgemeinen Formel III als Salz vorliegt. Aufgrund der unterschiedlichen physikalischen Eigenschaften, insbesondere der Löslichkeit, kann der vollständig methylierte, nicht zur Salzbildung befähigte Methoxycarbonsäuremethylester der allgemeinen Formel I leicht von den Methoxycarbonsäuren der allgemeinen Formel III abgetrennt werden. Letzterer kann anschließend beispielsweise, wie bereits gesagt, durch Zugabe von Säure, z.B. Salz- oder Schwefelsäure, bis zu einem geeignetem pH-Wert aus der Salzform in die Form der freien Säure überführt werden, die dann ebenfalls nach an sich bekannten Methoden, wie Filtration, Zentrifugation, Extraktion, Phasentrennung, gegebenenfalls auch nach Einengen oder teilweisem Einengen der Wasserphase, isoliert werden kann. Die Methoxycarbonsäuren der allgemeinen Formel III können aber z.B. auch durch Aussalzen oder durch Extraktion ihrer Salze gewonnen werden. Sie können im allgemeinen direkt, auch ohne Trocknung, in einen Folgeansatz eingesetzt werden.

Die gewünschten Methoxycarbonsäuremethylester der allgemeinen Formel I werden nach dem erfindungsgemäßen Verfahren in sehr hoher Reinheit erhalten, so daß aufwendige Reinigungsschritte im allgemeinen nicht notwendig sind. Ist die Verbindung der allgemeinen Formel I ein Feststoff, so ist es im allgemeinen ausreichend, das isolierte Produkt mit Wasser zu waschen und zu trocknen. Bei flüssigen Verbindungen der allgemeinen Formel I kann nach dem Abtrennen der Produktphase zum Entfernen von Wasser- und Salzresten eine einfache (Vakuum-)Destillation durchgeführt werden. Sollte für spezielle Verwendungsarten eine weitere Reinigung erforderlich sein, so kann diese nach an sich bekannten Methoden, beispielsweise durch Umkristallisieren, Destillieren, Sublimieren oder durch Chromatographieren erfolgen.

**Beispiel 1:**

188 g (1,00 mol) 6-Hydroxy-1-naphthoesäure und 115 g (0,57 mol) 6-Methoxy-1-naphthoesäure werden mit 154 g (2,75 mol) Kaliumhydroxid in 700 ml Wasser gelöst. Anschließend werden bei 40°C 130 g (1,03 mol) Dimethylsulfat zugetropft, wobei der pH-Wert durch Zugabe von KOH zwischen 10,6 - 11,0 gehalten wird. Bei fallendem pH-Wert werden sodann 210 g (1,67 mol) Dimethylsulfat zugetropft, wobei der pH-Wert durch Zugabe von KOH bei 8,8 - 9 gehalten wird. Man läßt nachrühren und stellt anschließend zur vollständigen Lösung der 6-Methoxy-1-naphthoesäure mit KOH auf pH = 12. Der rohe Ester wird abgetrennt, gewaschen und im Vakuum destilliert. Die wäßrige Phase wird sauer gestellt, die 6-Methoxy-1-naphthoesäure durch Filtration isoliert und dem nächsten Ansatz wieder zugesetzt (Auswaage: 115 g trocken).

Ausbeute: 208 g (96 %, bezogen auf eingesetzte 6-Hydroxy-1-naphthoesäure)
(die Gesamtausbeute an 6-Methoxy-1-naphthoesäuremethylester und 6-Methoxy-1-naphthoesäure, bezogen auf das eingesetzte Gemisch aus 6-Hydroxy- und 6-Methoxy-1-naphthoesäure, beträgt 98 %)
Reingehalt: >99 % (GC)
Schmelzpunkt: 44°C

**Vergleichsbeispiel 1 (gemäß Beispiel 1 der EP-B-200840):**

56 g (0,5 mol) Furan-2-carbonsäure und 270 g (2,5 mol) Anisol werden in 190 ml o-Dichlorbenzol gelöst. Anschließend versetzt man bei 40°C über 5 h mit 270 g (2,0 mol) wasserfreiem Aluminiumchlorid (30 Portionen zu 9 g). Nach erfolgter Zugabe verdünnt man mit 70 ml o-Dichlorbenzol und 100 g (0,93 mol) Anisol, rührt 3,5 h nach und hydrolysiert das Reaktionsgemisch durch 1-stündiges Rühren mit 1500 ml 6N Salzsäure. Nach Phasentrennung extrahiert man die wäßrige Phase mit 250 ml Essigsäure-isopropylester und erhält insgesamt ca. 950 g organische Phase, die ca. 0,12 mol 6-Methoxynaphthoesäure und 0,06 mol 6-Hydroxynaphthoesäure enthalten. Man extrahiert die organische Phase bei 60°C mit 400 ml gesättigter Kaliumhydrogencarbonat-Lösung, stellt die wäßrige Phase mit Salzsäure auf pH = 1 und löst die erhaltenen 6-Hydroxy/Methoxy-1-naphthoesäuren in 300 ml 1,2-Dichlorethan. Anschließend versetzt man mit 78 ml (0,82 mol) Dimethylsulfat, 108 g (0,78 mol) Kaliumcarbonat und 500 ml Aceton und läßt 3 h bei ca. 60°C rühren. Sodann werden die Lösungsmittel Aceton und Dichlorethan abdestilliert und der zurückbleibende 6-Methoxy-1-naphthoesäuremethylester wird im Vakuum destilliert.

Ausbeute: 34,5 g (77 %)
Reingehalt: 87 % (GC)
Schmelzpunkt: zähe Flüssigkeit, gelegentlich erstarrend (35 - 40°C)

Die folgende Übersicht stellt für die Herstellung von 6-Methoxy-1-naphthoesäuremethylester nochmals das erfindungsgemäße Verfahren denen des Standes der Technik gegenüber.

| | Erfindungs-gemäß Beispiel 1 | Stand der Technik EP-B-200840 | | US 4590290 |
|---|---|---|---|---|
| | | I[1) | II[1) | |
| Lösungs-mittel | Wasser | Aceton/ Wasser | Dichlorethan/ Aceton | Butyl-acetat |
| DMS-Menge[2) | 1,05 | 3,4 | 3,4 | 1,2 |
| Ausnutzungsgrad des Dimethyl-sulfats | 74 % | 20 % | 23 % | 66 % |
| Rein-ausbeute | 96 %[3) | 69 %[4) | 77 % | 78 % |
| Reinheit | >99 % | | 87 % | 93 % |

1)  I: Beispiel 4 aus der EP-B-200840;
    II: Vergleichsbeispiel 1 des vorliegenden Textes, in dem das Beispiel 1 der EP-B-200 840 nachgearbeitet wurde.

2)  Dimethylsulfat-Menge in Mol, bezogen auf die Ge-samtmolzahl zu methylierender Hydroxy- und Carboxy-gruppen im Ausgangsmaterial.

3)  Bezogen auf eingesetzte 6-Hydroxy-1-naphthoesäure; die Gesamtausbeute an 6-Methoxy-1-naphthoesäuremethylester und 6-Methoxy-1-naphthoesäure, bezogen auf das eingesetzte Gemisch aus 6-Hydroxy- und 6-Methoxy-1-naphthoesäure, beträgt 98 %.

4)  Rohausbeute, da Reinheit nicht angegeben.

**Beispiel 2:**

55,8 g (0,37 mol) 3-Hydroxy-4-methylbenzoesäure und 13,5 g (0,08 mol) 3-Methoxy-4-methylbenzoesäure werden mit 55,8 g (1,0 mol) Kaliumhydroxid in 300 ml Wasser gelöst. Anschließend werden bei 40 ° C 147 g (1,16 mol) Dimethylsulfat über ca. 3 h zugetropft, wobei der pH-Wert durch Zugabe von KOH bei 10,8 - 11 gehalten wird. Sodann wird noch 30 min nachgerührt, der Ester abgetrennt, mit Wasser gewaschen und im Vakuum getrocknet. Die wäßrige Phase wird sauer gestellt, die 3-Methoxy-4-methylbenzoesäure durch Filtration isoliert und dem nächsten Ansatz wieder zugesetzt (Auswaage: 13,5 g trocken).
Ausbeute:     64,3 g (97 %, bezogen auf eingesetzte 3-Hydroxy-4-methylbenzoesäure)

(die Gesamtausbeute an 3-Methoxy-4-methylbenzoesäuremethylester und 3-Methoxy-4-benzoesäure, bezogen auf das eingesetzte Gemisch aus 3-Hydroxy- und 3-Methoxy-4-methylbenzoesäure, beträgt 98 %)

Reingehalt: >99,5 % (GC)
Schmelzpunkt: 51°C

**Beispiel 3:**

Herstellung von 3,5-Dichlor-4-methoxybenzoesäuremethylester durch Methylierung eines Gemisches von 3,5-Dichlor-4-hydroxybenzoesäure und 3,5-Dichlor-4-methoxybenzoesäure

76,6 g (0,37 mol) 3,5-Dichlor-4-hydroxybenzoesäure und 28,7 g (0,13 mol) 3,5-Dichlor-4-methoxybenzoesäure (aus dem vorhergehenden Ansatz) werden mit 67 g 85%igem Kaliumhydroxid in 350 ml Wasser gelöst. Anschließend werden bei 40°C innerhalb von 3 Stunden 155 g (1,22 mol) Dimethylsulfat zugetropft, wobei der pH-Wert durch Zugabe von Kaliumhydroxid bei 11,5 gehalten wird. Nach Beendigung der Zugabe wird 30 Minuten nachgerührt, der ausgefallene 3,5-Dichlor-4-methoxybenzoesäuremethylester abgesaugt, mit Wasser gewaschen und im Vakuum getrocknet. Das wäßrige Filtrat wird sauer gestellt, die ausgefallene 3,5-Dichlor-4-methoxybenzoesäure durch Filtration isoliert und getrocknet. Es werden 23,9 g trockene 3,5-Dichlor-4-methoxybenzoesäure erhalten, die dem nächsten Ansatz wieder zugesetzt werden.

Ausbeute an 3,5-Dichlor-4-methoxybenzoesäuremethylester:
91,3 g (105 %, bezogen auf eingesetzte 3,5-Dichlor-4-hydroxybenzoesäure).
Die Geamtausbeute an 3,5-Dichlor-4-methoxybenzoesäuremethylester und 3,5-Dichlor-4-methoxybenzoesäure, bezogen auf das eingesetzte Gemisch aus 3,5-Dichlor-4-hydroxy- und -4-methoxybenzoesäure, beträgt 99 %.

Reingehalt: 97,2 % (GC)
Schmelzpunkt: 73 - 76°C

**Beispiel 4:**

Herstellung von 4-Methoxy-3-nitrobenzoesäuremethylester durch Methylierung eines Gemisches von 4-Hydroxy-3-nitrobenzoesäure und 4-Methoxy-3-nitrobenzoesäure.

42,1 g (0,23 mol) 4-Hydroxy-3-nitrobenzoesäure und 53,2 g (0,27 mol) 4-Methoxy-3-nitrobenzoesäure (aus dem vorhergehenden Ansatz) werden mit 60 g 85%igem Kaliumhydroxid in 550 ml Wasser gelöst. Anschließend werden bei 40°C innerhalb von 3 Stunden 155 g (1,22 mol) Dimethylsulfat zugetropft, wobei der pH-Wert durch Zugabe von 10 N KOH-Lösung bei oder über 11 gehalten wird. Nach Beendigung der Zugabe wird 30 Minuten nachgerührt, der pH-Wert auf 13,5 gestellt und der ausgefallene 4-Methoxy-3-nitrobenzoesäuremethylesterabgesaugt. Dieser wird mit Wasser gewaschen und im Vakuum getrocknet. Das wäßrige Filtrat wird sauer gestellt, die ausgefallene 4-Methoxy-3-nitrobenzoesäure durch Filtration isoliert und getrocknet. Es werden 53 g trockene 4-Methoxy-3-nitrobenzoesäure erhalten, die dem nächsten Ansatz wieder zugesetzt werden.

Ausbeute an 4-Methoxy-3-nitrobenzoesäuremethylester:
44,3 g (91,3 %, bezogen auf eingesetzte 4-Hydroxy-3-nitrobenzoesäure).
Die Gesamtausbeute an 4-Methoxy-3-nitrobenzoesäuremethylester und 4-Methoxy-3-nitrobenzoesäure, bezogen auf das eingesetzte Gemisch aus 4-Hydroxy- und 4-Methoxy-3-nitrobenzoesäure beträgt 95,8 %.

Reingehalt: 100 % (GC)
Schmelzpunkt: 107°C

**Patentansprüche**

1. Verfahren zur Herstellung von aromatischen Methoxycarbonsäuremethylestern der allgemeinen Formel I,

$$CH_3O-Ar-COOCH_3 \quad (I)$$

in der Ar für einen Phenylenrest oder für einen Naphthylenrest steht, wobei der Phenylenrest auch mono- oder disubstituiert und der Naphthylenrest auch mono-, di- oder trisubstituiert sein kann durch Halogenatome, $(C_1-C_4)$-Alkylgruppen, $(C_2-C_5)$-Alkenylgruppen, $(C_1-C_4)$-Alkoxygruppen, Benzyloxygruppen, Trifluormethylgruppen, Nitrogruppen, Hydroxymethylgruppen, Formylgruppen, Cyangruppen und (-

($C_1$-$C_4$)-Alkoxy)carbonylgruppen,
durch Umsetzung von aromatischen Hydroxycarbonsäuren der allgemeinen Formel II

HO-Ar-COOH     (II)

im Gemisch mit aromatischen Methoxycarbonsäuren der allgemeinen Formel III,

$CH_3$O-Ar-COOH     (III)

wobei Ar in II und III wie für I angegeben definiert ist, mit Dimethylsulfat in Gegenwart einer Base, dadurch gekennzeichnet, daß man die Umsetzung in Wasser durchführt und daß man die 0,8 bis 1,7fache molare Menge an Dimethylsulfat, bezogen auf die Gesamtmolzahl zu methylierender Hydroxy- und Carboxygruppen in den Ausgangssubstanzen, einsetzt.

2.  Verfahren gemäß Anspruch 1, dadurch gekennzeichnet, daß man eine Mischung umsetzt, die pro Mol Hydroxycarbonsäure der allgemeinen Formel II bis zu 2 mol der zugehörigen Methoxycarbonsäure der allgemeinen Formel III enthält.

3.  Verfahren gemäß Anspruch 1 und/oder 2, dadurch gekennzeichnet, daß die in dem umzusetzenden Carbonsäure-Gemisch enthaltene Methoxycarbonsäure der allgemeinen Formel III vollständig oder teilweise aus einer oder mehreren vorherigen Umsetzungen gemäß Anspruch 1 und/oder 2 stammt.

4.  Verfahren gemäß einem oder mehren der Ansprüche 1 bis 3, dadurch gekennzeichnet, daß man die Umsetzung zwischen 10° und 90°C, bevorzugt zwischen Raumtemperatur und 70°C, besonders bevorzugt zwischen 30° und 60°C, durchführt.

5.  Verfahren gemäß einem oder mehreren der Ansprüche 1 bis 4, dadurch gekennzeichnet, daß man die Umsetzung in Gegenwart eines Alkalimetallhydroxids, bevorzugt in Gegenwart von Natrium- oder Kaliumhydroxid, durchführt.

6.  Verfahren gemäß einem oder mehreren der Ansprüche 1 bis 5, dadurch gekennzeichnet, daß man 6-Hydroxy-1-naphthoesäure im Gemisch mit 6-Methoxy-1-naphthoesäure zu 6-Methoxy-1-naphthoesäuremethylester umsetzt.

7.  Verfahren gemäß Anspruch 6, dadurch gekennzeichnet, daß man eine Mischung umsetzt, die pro Mol 6-Hydroxy-1-naphthoesäure 0,1 bis 1 mol, bevorzugt 0,3 bis 0,8 mol, 6-Methoxy-1-naphthoesäure enthält.

8.  Verfahren gemäß Anspruch 6 und/oder 7, dadurch gekennzeichnet, daß man die 1,0 bis 1,3fache, bevorzugt die 1,0 bis 1,1fache molare Menge an Dimethylsulfat, bezogen auf die Gesamtmolzahl zu methylierender Hydroxy- und Carboxygruppen in der 6-Hydroxy- und der 6-Methoxy-1-naphthoesäure, einsetzt.

9.  Verfahren gemäß einem oder mehreren der Ansprüche 1 bis 5, dadurch gekennzeichnet, daß man 3-Hydroxy-4-methylbenzoesäure im Gemisch mit 3-Methoxy-4-methylbenzoesäure zu 3-Methoxy-4-methylbenzoesäuremethylester umsetzt.

10. Verfahren gemäß Anspruch 9, dadurch gekennzeichnet, daß man eine Mischung umsetzt, die pro Mol 3-Hydroxy-4-methylbenzoesäure 0,05 bis 1 mol, bevorzugt 0,1 bis 0,4 mol, 3-Methoxy-4-methylbenzoesäure enthält.